(19) Europäisches Patentamt
European Patent Office
Office européen des brevets

(11) Veröffentlichungsnummer: **0 038 005**
**A1**

(12) # EUROPÄISCHE PATENTANMELDUNG

(21) Anmeldenummer: **81102594.9**

(22) Anmeldetag: **07.04.81**

(51) Int. Cl.³: **C 07 C 125/073**, C 07 C 125/077

(30) Priorität: **11.04.80 DE 3013907**

(43) Veröffentlichungstag der Anmeldung: **21.10.81**
**Patentblatt 81/42**

(84) Benannte Vertragsstaaten: **BE DE FR GB IT NL**

(71) Anmelder: **BASF Aktiengesellschaft,**
**Carl-Bosch-Strasse 38, D-6700 Ludwigshafen (DE)**

(72) Erfinder: **Merger, Franz, Dr., Max-Slevogt-Strasse 25,**
**D-6710 Frankenthal (DE)**
Erfinder: **Nestler, Gerhard, Dr., Van-Leyden-Strasse 17,**
**D-6700 Ludwigshafen (DE)**

(54) **Verfahren zur Herstellung von Methylen-bis-phenylcarbaminsäure-estern und Polymethylen-polyphenylcarbaminsäureestern.**

(57) Verfahren zur Herstellung von Methylen-bis-phenylcarbaminsäureestern und Polymethylenpolyphenylcarbaminsäureestern durch Umsetzung von N-Phenylcarbaminsäureestern mit Formaldehyd, Formaldehydderivaten oder Formaldehyd abgebenden Verbindungen in Gegenwart von Carbonsäuren, deren pKs-Wert kleiner als 4 ist.

EP 0 038 005 A1



COMPLETE DOCUMENT

Verfahren zur Herstellung von Methylen-bis-phenylcarbaminsäureestern und Polymethylen-polyphenylcarbaminsäureestern

Die Erfindung betrifft ein neues Verfahren zur Herstellung von Methylen-bis-phenylcarbaminsäurestern und davon abgeleiteten höheren homologen Polymethylen-polyphenylcarbaminsäureestern durch Umsetzung von N-Phenylcarbaminsäureestern mit Formaldehyd oder formaldehydabspaltenden Verbindungen in Gegenwart von Carbonsäuren.

Methylen-bis-phenylcarbaminsäureester und davon abgeleitete höhere Homologe sind wertvolle Ausgangsprodukte für die Herstellung von Methylen-bis-phenylisocyanaten und den entsprechenden Polymethylenpolyphenylisocyanaten, die bekanntlich für die Herstellung von Polyurethanen Verwendung finden (DE-OS 2 635 490). Die im Handel erhältlichen derartigen Isocyanate werden allgemein durch Phosgenierung der bei der Kondensation von Anilin mit Formaldehyd in Gegenwart von wäßrigen Säuren erhältlichen Amine hergestellt.

Die Herstellung der Methylen-bis-phenylcarbaminsäureester erfolgt durch Umsetzung derartiger Anilin-Formaldehyd-Kondensationsprodukte mit Chlorameisensäureestern in Gegenwart von Basen oder durch Umsetzung der entsprechenden Isocyanate mit Alkoholen.

Diese Verfahren haben den Nachteil, daß die Umsetzung mit dem stark toxischen Phosgen aus Sicherheitsgründen eine aufwendige Technik erfordert und die Abtrennung der bei den einzelnen Umsetzungen einerseits benötigten und andererseits als Nebenprodukt anfallenden Säuren die Umwelt stark belastet.

Hee/Bl

Methylen-bis-phenylcarbaminsäureester lassen sich auch durch Umsetzung von Methylen-bis-nitrophenyl mit Alkoholen und Kohlenmonoxid herstellen (s. DE-AS 1 568 044). Da die Darstellung der hierfür benötigten Nitroverbindungen große Schwierigkeiten bereitet, hat dieses Verfahren keine technische Bedeutung erlangt.

In der US-PS 2 946 768 wird ein Verfahren beschrieben, bei dem man Phenylcarbaminsäureester mit Formaldehyd oder Formaldehyd abgebenden Verbindungen in Gegenwart von Säuren erhitzt. Über die dabei erhaltenen Kondensationsprodukte wird gesagt, daß sie C-C-Verknüpfungen aufweisen, wobei aber auch N-C-Verknüpfungen nicht ausgeschlossen werden (s. Spalte 2, Zeilen 65 - 70).

In der DE-OS 2 832 379 wird angegeben, daß mit früher nicht bekannten analytischen Methoden nachgewiesen werden konnte, daß bei dem Verfahren der US-PS 2 946 768 Formaldehyd zur Reaktion am Stickstoffatom des Carbaminsäureesters neigt und daher 15 bis 50 Gew.% an unerwünschten N-C-verknüpften Produkten entstehen. Durch ein analog Beispiel 2 der US-PS 2 946 768 durchgeführtes Vergleichsbeispiel (s. Beispiel 1) wird dieses Ergebnis bestätigt. Außerdem werden noch andere Nebenprodukte gebildet, beispielsweise entstehen durch saure Hydrolyse des Carbaminsäureesters Amine (s. DE-OS 28 32 379, Seite 3).

Da es kein Verfahren zur Abtrennung der über das Stickstoffatom verknüpften Produkte, die bei der Pyrolyse keine Isocyanaten liefern, gibt, sind die nach der US-PS 2 946 768 hergestellten Reaktionsgemische zur Herstellung von Isocyanaten ungeeignet. Dieses Verfahren ist daher in technischer und ökonomischer Hinsicht unbefriedigend.

In der DE-OS 28 32 379 wird nun ein Verfahren zur Umlagerung dieser unerwünschten Nebenprodukte zu Methylen-bis-phenylcarbaminsäurestern und davon abgeleiteten höheren homologen Polymethylenpolyphenylcarbaminsäureestern beschrieben. Es besteht darin, daß man die nach dem Verfahren der US-PS 2 946 768 hergestellten Reaktionsgemische unter praktisch wasserfreien Bedingungen bei 50 bis 170°C mit starken Protonensäuren oder Lewissäuren umsetzt.

Der Nachteil dieses technisch schwierigen und zweistufigen Verfahrens besteht darin, daß zunächst nach dem Verfahren der US-PS 2 946 768 aus Phenylcarbaminsäureestern und Formalin in Gegenwart erheblicher Mengen wäßriger Säuren ein Kondensationsprodukt hergestellt wird, dieses von der Säure befreit und getrocknet werden muß und schließlich unter wasserfreien Bedingungen erneut mit großen Mengen an Säure, die nach Ablauf der Reaktion ebenfalls wieder vollständig zu entfernen sind, umgesetzt wird. Die Abwasserbelastung durch die großen Säuremengen und die teilweise gebildeten Nebenprodukte, z.B. durch die bei der sauren Hydrolyse entstandenen Amine, ist ein ernstes Problem.

In der US-PS 4 162 362 wird die einstufige Kondensation des Phenylcarbaminsäureethylesters mit Formaldehyd oder formaldehydspaltenden Verbindungen in Gegenwart von Sulfonsäuren beschrieben. Dabei werden relativ große Mengen Sulfonsäure, bezogen auf den Carbaminsäureester, benötigt. Da das Reaktionsprodukt säurefrei gewaschen werden muß, ist aufgrund der hohen Viskosität des Kondensationsproduktes die Verwendung eines Lösungsmittels und ein aufwendiger Aufarbeitungsprozeß erforderlich. Die durch die Rückführung bzw. Beseitigung der Sulfonsäure bedingte Umweltbelastung ist beträchtlich.

Es wurde nun gefunden, daß man bei der Herstellung von Methylen-bis-phenylcarbaminsäureestern und Polymethylen-polyphenylcarbaminsäureestern durch Umsetzung von N-Phenylcarbaminsäureestern mit Formaldehyd, Formaldehydderivaten oder Formaldehyd abgebenden Verbindungen die genannten Nachteile vermeidet, wenn man die Umsetzung in Gegenwart einer Carbonsäure vornimmt, deren pKs-Wert kleiner als 4 ist.

Im Gegensatz zu den oben genannten bekannten Verfahren, bei denen die Abtrennung der verwendeten Säuren eine umständliche, aufwendige und umweltbelastende Arbeitsweise erforderlich macht, können die bei dem erfindungsgemäßen Verfahren verwendeten Carbonsäuren einfach destillativ abgetrennt werden. Die Rückführung der Carbonsäuren in den Prozeß ist daher äußerst einfach.

Das Reaktionsschema kann für den Fall der Bildung des Methylen-bis-(4-phenylcarbaminsäureesters) aus dem N-Phenylcarbaminsäuremethylester und Formaldehyd durch folgende Formeln wiedergegeben werden.

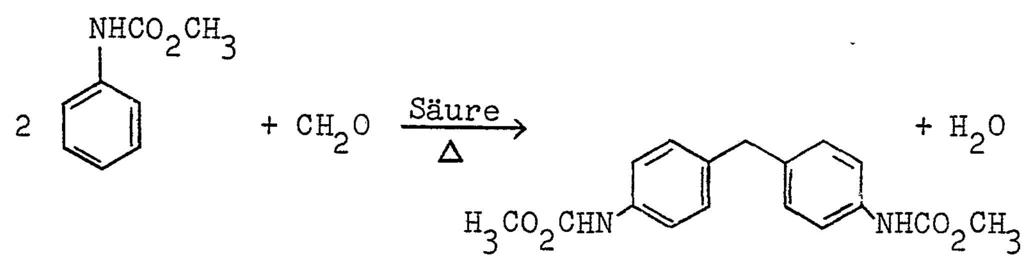


Bei dem erfindungsgemäßen Verfahren werden gleichzeitig auch höhere homologe Polymethylen-polyphenylcarbaminsäureester, das sind 3- und mehrere durch Methylenbrücken miteinander verbundene Benzolringe enthaltende Carbaminsäureester, gebildet, da Formaldehyd in untergeordentem Maße auch mit bereits gebildetem Methylen-bis-phenylcarbaminsäureester reagiert.

Als N-Phenylcarbaminsäureester kommen z.B. Verbindungen der Formel

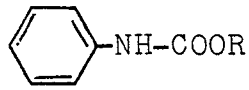

in Betracht, in der R ein Alkylrest mit 1 bis 3 C-Atomen bedeutet und der Phenylrest in den o- und/oder m-Stellungen beispielsweise durch Methyl- oder Methoxygruppen oder durch Halogenatome wie Chlor- oder Bromatome, substituiert sein kann.

Geeignete N-Phenylcarbaminsäurester sind beispielsweise N-Phenylcarbaminsäuremethyl-, äthyl- oder -propylester, N-o-Tolylcarbaminsäuremethyl- oder -ethylester, N-2.6-Dimethylphenylcarbaminsäuremethylester oder N-o-Chlorphenylcarbaminsäureethylester.

Anstelle von Formaldehyd können auch Formaldehyd abspaltende Verbindungen wie Paraformaldehyd und Trioxan, oder spezielle Formaldehydderivate verwendet werden. Solche speziellen Formaldehydverbindungen sind z.B. Verbindungen der allgemeinen Formel $X-CH_2-X$, in der X die Reste OR, SR, OCOR und R Alkylreste mit vorzugsweise 1 bis 3 C-Atomen bedeuten. Bei Verwendung von Acetalen werden vorzugsweise solche gewählt, in denen der Alkylrest mit dem Alkanol übereinstimmt, der dem Carbaminsäureester zugrundeliegt. Besonders vorteilhaft ist Dimethylformal, da es sich sehr leicht und wirtschaftlich aus wäßrigem Formaldehyd und Methanol gewinnen läßt.

Man führt die erfindungsgemäße Umsetzung im allgemeinen bei Temperaturen bis 160°C, vorzugsweise bei 30 bis 160°C, insbesondere 40 bis 140°C durch.

Das Molverhältnis Formaldehyd : Carbaminsäureester liegt zweckmäßig bei 1 : 0.5 bis 1 : 10, vorzugsweise bei 1 : 1.5 bis 1 : 3. Sollen jedoch hauptsächlich Methylen-bis-phenylcarbaminsäureester hergestellt werden, so verwendet man vorzugsweise ein Verhältnis von 1 : 4 bis 1 : 8.

Als Carbonsäuren mit pKs-Werten kleiner als 4, die man z.B. in Mengen von 1 bis 300, vorzugsweise 10 bis 200 Gew.%, bezogen auf den Carbaminsäureester, anwendet, kommen z.B. Ameisensäure, Dichloressigsäure, Trichloressigsäure, Trifluoressigsäure, Oxalsäure oder Gemische dieser Säuren in Betracht. Die Umsetzung, die etwa nach 1 bis 20 Stunden beendet ist, wird im allgemeinen so durchgeführt, daß man entweder zu einem Gemisch aus dem Carbaminsäureester und der Säure unter Rühren bei der Reaktionstemperatur Formaldehyd oder die entsprechende Verbindung langsam zugibt oder daß man ein Gemisch aus dem Carbaminsäureester, der Formaldehydkomponente und der Säure unter Rühren erhitzt und die Gemische die entsprechende Zeit auf der Reaktionstemperatur hält. Mitunter ist es auch sinnvoll, bei tieferer Temperatur die Umsetzung zu beginnen und anschließend die Temperatur stufenweise zu erhöhen. Die Isolierung des Reaktionsproduktes erfolgt nach herkömmlichen Methoden, z.B. durch destillative Abtrennung der Carbonsäure, des gegebenenfalls vorhandenen Lösungsmittels und unumgesetzten Ausgangsproduktes.

Die Kondensation kann sowohl ohne als auch in Gegenwart eines Lösungsmittels, wie Benzol, Methylcyclohexan, Essigsäure, Methanol, Methylacetat, Nitrobenzol, Chlorbenzol, Dichlorbenzol oder chlorierte aliphatische Kohlenwasserstoffe durchgeführt werden. Falls ein Lösungsmittel notwendig ist, verwendet man jedoch vorzugsweise die als Katalysator eingesetzte Carbonsäure als Lösungsmittel.

Die Umsetzungen können sowohl drucklos als auch unter Druck durchgeführt werden.

Beispiel 1 (Vergleichsbeispiel)

Analog Beispiel 2 der DE-PS 1 042 891 wird ein Gemisch aus 183 Teilen Phenylcarbaminsäuremethylester, 500 ml Wasser und 86 Teilen Formalin (30%ig) unter Rühren auf 100°C erwärmt, dann gibt man 100 ml konzentrierte Salzsäure hinzu. Das Reaktionsgemisch wird anschließend 20 Stunden bei 100°C gerührt. Nach Beendigung der Umsetzung wird die wäßrige Phase abgetrennt und das Reaktionsprodukt dreimal mit heißem Wasser gewaschen. Anschließend wird unumgesetztes Ausgangsprodukt im Vakuum abdestilliert. Der Rückstand wird mittels Hochdruck-Flüssigkeits-Chromatographie (HPLC) analysiert. Er enthält 50 % Methylen-bis-phenylcarbaminsäuremethylester, 9 % Dreikernprodukt, 16 % N-C-verknüpftes Zweikernprodukt und 10 % N-C-verknüpftes Dreikernprodukt. Der Rest besteht aus nicht näher identifizierten höherkernigen Verbindungen.

Beispiel 2

In einem Rührreaktor wird ein Gemisch aus 151 Teilen Phenylcarbaminsäuremethylester, 15 Teilen Trioxan und 200 Teilen Ameisensäure auf 100°C erhitzt und 20 Stunden bei dieser Temperatur gerührt. Nach Beendigung der Umsetzung wird im Vakuum destilliert. Man erhält 145 Teile eines Destillationsrückstandes, der entsprechend der HPLC-Analyse aus 65 % Methylen-bis-phenylcarbaminsäuremethylester, 22 % 3-Kernprodukt und 13 % höherkernigen Produkten besteht.

Beispiel 3

In einem Rührreaktor wird ein Gemisch aus 151 Teilen Phenylcarbaminsäuremethylester, 66 Teilen Diacetoxymethan, 50 Teilen Oxalsäure unter Rühren auf 110°C erhitzt und 20 Stunden bei dieser Temperatur gerührt. Nach Beendigung der Umsetzung wird im Vakuum nicht umgesetztes Ausgangsmaterial abdestilliert. Man erhält 124 Teile eines Destillationsrückstandes, der entsprechend der HPLC-Analyse aus 68 % Methylen-bis-phenylcarbaminsäuremethylester, 17 % 3-Kernprodukt und 25 % höherkernigen Produkten besteht.

Beispiel 4

In einem Rührreaktor wird ein Gemisch aus 151 Teilen Phenylcarbaminsäuremethylester, 50 Teilen 60%ige wäßrige Formaldehydlösung und 300 Teilen Ameisensäure auf 100°C erhitzt und 20 Stunden bei dieser Temperatur gerührt. Nach Beendigung der Umsetzung wird im Vakuum destilliert. Man erhält 138 Teile eines Destillationsrückstandes, der entsprechend der HPLC-Analyse aus 58 % Methylen-bis-phenylcarbaminsäuremethylester, 22 % 3-Kernprodukt und 20 % höherkernigen Produkten besteht.

Beispiel 5

In einem Rührreaktor wird ein Gemisch aus 151 Teilen Phenylcarbaminsäuremethylester, 15 Teilen Paraformaldehyd und 200 Teilen Trifluoressigsäure unter Rühren auf 100°C erhitzt und 20 Stunden bei dieser Temperatur gerührt. Nach Beendigung der Umsetzung wird im Vakuum die Säure und nicht umgesetztes Ausgangsmaterial abdestilliert. Man erhält 130 Teile eines Destillationsrückstandes, der entsprechend der HPLC-Analyse aus 51 % Methylen-bis-phenylcarbaminsäuremethylester, 19 % 3-Kernprodukt und 30 % höherkettigen Produkten besteht.

Patentansprüche

1. Verfahren zur Herstellung von Methylen-bis-phenylcarbaminsäureestern und Polymethylenpolyphenylcarbaminsäureestern durch Umsetzung von N-Phenylcarbaminsäureestern mit Formaldehyd, Formaldehydderivaten oder Formaldehyd abgebenden Verbindungen dadurch gekennzeichnet, daß man die Umsetzung in Gegenwart von Carbonsäuren vornimmt, deren pKs-Wert kleiner als 4 ist.

2. Verfahren nach Anspruch 1, dadurch gekennzeichnet, daß man die Umsetzung bei 30 bis 160°C durchführt.

3. Verfahren nach Anspruch 1, dadurch gekennzeichnet, daß man als Carbonsäuren mit pKs-Werten kleiner als 4 Ameisensäure, Oxalsäure, Dichloressigsäure, Trichloressigsäure oder Trifluoressigsäure verwendet.

Europäisches Patentamt

# EUROPÄISCHER RECHERCHENBERICHT

0038005
Nummer der Anmeldung
EP 81 10 2594.9

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| P,X | DE - A1 - 2 950 386 (MITSUI TOATSU CHEMICALS)<br>* Anspruch 6 * | 1-3 | C 07 C 125/073<br>C 07 C 125/077 |
| P | & FR - A - 2 444 057 | | |
| P | & NL - A - 7 908 982 | | |
| P | & GB - A - 2 044 252 | | |
| P | & JP - A - 55 081850 | | |
| P | & JP - A - 55 081851<br>-- | | RECHERCHIERTE SACHGEBIETE (Int. Cl.³) |
| P,A | Patents Abstracts of Japan<br>Band 4, Nr. 124, 2. September 1980,<br>Seite 107C23 | | C 07 C 125/073<br>C 07 C 125/077 |
| P | & JP - A - 55 - 79358<br>-- | | |
| D,A | US - A - 4 162 362 (ATLANTIC RICHFIELD)<br>* vollständig *<br>& DE - A - 2 931 473<br>-- | | |
| D,A | DE - C - 1 042 891 (BAYER)<br>* vollständig *<br>--- | | KATEGORIE DER GENANNTEN DOKUMENTE |

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 01-07-1981 | BREW |